# EUROPEAN PATENT SPECIFICATION

(11) **EP 3 920 907 B1**
(45) Date of publication and mention of the grant of the patent: **09.12.2026**
(21) Application number: 20891077.8
(22) Date of filing: 08.04.2020
(51) Int. Cl.: A61K 31/353, A61K 31/355, A61K 31/575, A61K 31/01, A61K 45/06, A61P 25/00

(54) **A COMPOSITION COMPRISING VITAMIN E TOCOTRIENOLS, SQUALENES AND PHYTOSTEROLS FOR MANAGING CEREBRAL AUTOSOMAL-DOMINANT ARTERIOPATHY WITH SUBCORTICAL INFARCTS AND LEUKOENCEPHALOPATHY (CADASIL)**
ZUSAMMENSETZUNG MIT VITAMIN-E-TOCOTRIENOL ZUR BEHANDLUNG VON ZEREBRALER AUTOSOMAL DOMINANTER ARTERIOPATHIE MIT SUBKORTIKALEN INFARKTEN UND LEUKOENZEPHALOPATHIE (CADASIL)
COMPOSITION COMPRENANT DES TOCOTRIÉNOLS DE VITAMINE E, SQUALÈNES ET PHYTOSTEROLS POUR GÉRER L'ARTÉRIOPATHIE CÉRÉBRALE AUTOSOMIQUE DOMINANTE AVEC INFARCTUS SOUS-CORTICAUX ET LEUCOENCÉPHALOPATHIE (CADASIL)

(30) Priority: 19.11.2019 MY PI2019006779
(43) Date of publication of application: 15.12.2021
(73) Proprietor: Hovid Berhad, 30010 Ipoh, Perak (MY)
(72) Inventor: YUEN, Kah Hay, 30010 Ipoh Perak (MY); WONG, Jia Woei, 30010 Ipoh Perak (MY); HO, David Sue San, 30010 Ipoh Perak (MY); FUNG, Wai Yee, 30010 Ipoh Perak (MY)
(74) Representative: Nederlandsch Octrooibureau
(86) International application number: PCT/MY2020/050020
(87) International publication number: WO 2021/101367

(56) References cited:
- WO-A1-2010/151348
- WO-A1-2013/185022
- WO-A1-2017/204618
- JIAO YANG ET AL: "Neuroprotective Effects of Tocovid Pretreatment in a Mouse Stroke Model", JOURNAL OF STROKE AND CEREBROVASCULAR DISEASES, vol. 27, no. 8, August 2018 (2018-08-01), US, pages 2166 - 2174, XP055979740, ISSN: 1052-3057, DOI: 10.1016/j.jstrokecerebrovasdis.2018.03.014
- ANONYMOUS: "Tocovid SupraBio, Your Natural Shield! | Hovid Berhad", 1 June 2011 (2011-06-01), XP055241262, Retrieved from the Internet <URL:https://hovid.wordpress.com/2011/06/01/tocovid-suprabio-your-natural-shield/comment-page-2/> [retrieved on 20160114]
- FERRANTE ELISA A ET AL: "CADASIL: new advances in basic science and clinical perspectives.", CURRENT OPINION IN HEMATOLOGY 05 2019, vol. 26, no. 3, May 2019 (2019-05-01), pages 193 - 198, XP009540488, ISSN: 1531-7048
- CAMPOLO JONICA ET AL: "Is the Oxidant/Antioxidant Status Altered in CADASIL Patients?", PLOS ONE, vol. 8, no. 6, 14 June 2013 (2013-06-14), US, pages e67077, XP093239751, ISSN: 1932-6203, DOI: 10.1371/journal.pone.0067077
- CHABRIAT, H ET AL.: "Neuropsychiatric manifestations in CADASIL", DIALOGUES IN CLINICAL NEUROSCIENCE, vol. 9, no. 2, 2007, pages 199 - 208, XP055827837
- GOPALAN, Y. ET AL.: "Clinical Investigation of the Protective Effects of Palm Vitamin E Tocotrienols on Brain White Matter", STROKE, vol. 45, no. 5, 2014, pages 1422 - 1428, XP055445202, DOI: 10.1161/STROKEAHA.113.004449
- SHANG JINGWEI, YAN HONGJING, JIAO YANG, OHTA YASUYUKI, LIU XIA, LI XIANGHONG, MORIHARA RYUTA, NAKANO YUMIKO, FUKUI YUSUKE, SHI XIA: "Therapeutic Effects of Pretreatment with Tocovid on Oxidative Stress in Postischemic Mice Brain", JOURNAL OF STROKE AND CEREBROVASCULAR DISEASES, vol. 27, no. 8, 2018, pages 2096 - 2105, XP055827839
- LING, S. ET AL.: "Tocomin tocotrienol/tocopherol complex. A potent natural neuroprotective vitamin", NUTRACOS, vol. 8, no. 5, 30 November 2008 (2008-11-30), pages 22 - 24, XP009530306, ISSN: 1720-4011

## Description

### Field of Invention

The present invention relates to therapeutic management of a hereditary stroke disorder. More particularly, the invention relates to a composition comprising vitamin E tocotrienols, squalenes and phytosterols for use managing the symptoms of Cerebral Autosomal-Dominant Arteriopathy with Subcortical Infarcts and Leukoencephalopathy (CADASIL)

### Background of the Invention

Cerebral Autosomal-Dominant Arteriopathy with Subcortical Infarcts and Leukoencephalopathy (CADASIL) was genetically defined in 1993. It is a heritable small-vessel disease caused by mutations in NOTCH3 gene located on chromosome 19, which is normally expressed in vascular smooth muscle cells and pericytes. CADASIL is an autosomal dominant inheritance disorder, meaning that one parent carries and passes on the defective gene. While most individuals with CADASIL have a family history of the disorder, there are a few exceptional cases, where the mutation of the NOTCH3 gene occurred randomly without having been transmitted by one of the parents.

The genetic mutation in CADASIL alters the muscular walls in small arteries. Cells in the smooth muscle layer of the arteriolar walls gradually degenerate, and are replaced by fibrous connective tissue. This leads to progressive wall thickening, and luminal narrowing, resulting in decreased blood flow. Small branches of long arteries penetrating deep into the white matter of the brain are generally affected. This microvascular changes and dysfunction will result in hypoperfusion of the brain especially the white matter regions, leading to small lacunar infarcts in the white matter and in deep parts of the grey matter (the basal ganglia). Despite the conspicuous early absence of vascular risk factors such as hypertension and hypercholesterolemia, CADASIL patients suffer recurrent acute ischemic events, almost exclusively lacunar infarcts involving subcortical white matter, deep gray matter nuclei and brain stem, diffuse white matter demyelination and axonal loss (Ayata, 2010). Physiological studies have shown an age-dependent reduction in resting cerebral blood flow, while hypoperfusion was spatially limited to white matter regions that showed leukoaraiosis (Ayata, 2010).

CADASIL is slowly progressive and up to 50% may suffer several transient ischemic attacks (TIAs) or strokes, with considerable variations between individuals (Alves *et al.,* 2008). CADASIL, due to its onset in young patients without concomitant cognitive disorders and other confounding risk factors of vascular etiologies such as hypercholesterolemia, hypertension and diabetes, can be considered the pure form of subcortical ischemic dementia. Dementia is a common complication of subcortical ischemic vascular disease (SIVD) and is present in about 80% of CADASIL patients at the time of death (André, 2010). SIVD encompasses 3 basic pathological entities: small vessel disease, lacunar infarct and ischemic white matter lesions (WML). WML has been noted to be an independent factor in cognitive decline, with the most impaired domains being executive, attentional and memory retrieval mechanisms (Alves *et al.,* 2008). Cognitive impairment and dementia correlate with the extent of cumulative subcortical pathology, in particular the lacunar infarct burden and brain atrophy (Ayata, 2010; Viswanathan *et al.,* 2007; Liem *et al.,* 2007). Manifestations of executive dysfunction (almost 100% between 35 and 50 years of age) and attentional deficits (69%) are among the earliest cognitive changes (Buffon *et al.,* 2006; André, 2010). CADASIL patients suffer from ischemic episodes, cognitive decline, migraine and psychiatric problems, with highly variable onset and severity (Alves *et al.,* 2008). In the final stages, individuals are bedridden, apathetic and totally dependent. Time to death is also highly variable in 10 to 30 years, from accumulation of morbidities and clinical complications related to infection and immobility (Opherk, 2004; André, 2010). WO 2017/204618 (ATTEST RESEARCH SDN BHD), 30 November 2017, discloses a composition comprising tocotrienols, tocopherols and B vitamins for preventing or mitigating dementia by preventing or delaying the progression of white matter lesions. Ferrante EA et al, 2019, Current Opinion in Hematology, 26(3):193-198 deals with the clinical perspectives of CADASIL. It teaches that the predominant clinical features include migraines with aura, recurrent strokes of ischemic nature, transient ischemic attacks, progressive white matter degeneration, memory loss, debilitating dementia and disability and multiple psychiatric symptoms.

There is currently no treatment to stop the progression or development of the genetically inherited neurodegenerative disease. The disease management consists of drug therapy for the symptomatic migraines, epilepsy and psychiatric problems such as depression. Patients are advised to quit smoking and treated with aspirin to reduce risk of stroke, while other vascular risk factors such as diabetes, hypertension, hyperlipidemia, are aggressively treated. Patients with significant cognitive deficit are treated with centrally acting cholinesterase inhibitors or other drugs for neurodegenerative disorders. However, a clinical study using a cholinesterase inhibitor, donepezil (Dichgan *et al.,* 2008) in CADASIL failed to show any treatment effect in any of the cognitive and executive function assessments when compared to placebo.

Thus, there is a need for an effective therapeutic management of CADASIL, especially in mitigating the undesirable alterations of the vasculature and reducing the risk of ischemic events, which is the main cause to debilitating cognitive decline and accumulating morbidities in CADASIL patients. There is no animal model that can fully recapitulate human CADASIL phenotype. Hence, a neuroprotective agent for reducing accumulation of cerebral lesions is a promising approach for impeding CADASIL disease progression.

### Summary of the Invention

The primary object of the invention is to provide a composition comprising Vitamin E tocotrienols, squalenes and phytosterols for use in the effective therapeutic management of Cerebral Autosomal-Dominant Arteriopathy with Subcortical Infarcts and Leukoencephalopathy (CADASIL) Particularly, the composition comprises alpha-tocotrienol, gamma-tocotrienol, delta-tocotrienol or alpha-tocopherol, or any combination thereof.

Another object of the invention is to provide a composition for use in effectively mitigating the negative effects of cerebrovasculature alterations and debilitating symptoms in subjects suffering from CADASIL including abnormal brain lesions, cognitive impairment, memory deterioration, dementia, occurrence of ischemic events, occurrence of disability, multiple strokes, migraine headaches, seizures, vision problems and/or psychiatric problems such as depression, apathy and mood disturbances.

At least one of the preceding objects is met, in whole or in part, by the present invention, in which the embodiment of the present invention describes a composition for use in mitigating debilitating effects of Cerebral Autosomal-Dominant Arteriopathy with Subcortical Infarcts and Leukoencephalopathy (CADASIL), wherein the composition comprises vitamin E tocotrienols in a mixture of squalenes, phytosterols and pharmaceutically acceptable excipients.

Preferably, the debilitating effects can be any one or any combination of abnormal brain lesions, cognitive impairment, dementia, memory deterioration, occurrence of ischemic events, occurrence of disability, migraine headaches, multiple strokes, seizures, vision problems and psychiatric problems such as depression, apathy and mood disturbances.

In the preferred embodiment of the invention, vitamin E tocotrienols is any one or any combination of alpha-tocotrienol, gamma-tocotrienol, beta- tocotrienol, and delta-tocotrienol. The composition may further comprise an alpha-tocopherol. Preferably, the vitamin E tocotrienols and tocopherols are derived from plants selected from the group consisting of palm oil, rice bran oil, barley, oat, rye, wheat germ and annatto. Vitamin E tocotrienols may present at a concentration ranging from 10 to 40 % by weight of the composition.

More particularly, the alpha-tocotrienol is present at a concentration ranging from 3 to 20 % by weight of the composition. Beta-tocotrienol may constitute 0.7 to 3.0 % by weight of the composition. The gamma-tocotrienol and delta-tocotrienol may present in the composition at a concentration ranging from 6 to 30 % and 1.5 to 12 % by weight of the composition, respectively. Alpha-tocopherol may present in the composition at a concentration ranging from 3 to 15 % by weight of the composition.

Preferably, squalene used in the composition is derived from plants. Squalene may present at a concentration ranging from 2.5 to 10 % by weight of the composition.

Pursuant in the preferred embodiment of the invention, pharmaceutically acceptable excipients such as plant-based oil, water-based emulsifiers, oil-based emulsifiers, co-emulsifiers, antioxidants, and suspending agents are used. Preferably, the pharmaceutically acceptable excipient is present at a concentration ranging from 0.1 to 50 % by weight of the composition.

### Detailed Description of the Invention

One skilled in the art will readily appreciate that the present invention is well adapted to carry out the objects and obtain the ends and advantages mentioned, as well as those inherent therein. The embodiment described herein is not intended as limitations on the scope of the invention.

The present invention discloses a composition for use in mitigating or managing effects of Cerebral Autosomal-Dominant Arteriopathy with Subcortical Infarcts and Leukoencephalopathy (CADASIL), wherein the composition comprises vitamin E tocotrienols in a mixture of squalenes, phytosterols and pharmaceutically acceptable excipients. Particularly, the composition can be used in managing or mitigating manifestations of CADASIL including cognitive impairment, memory deterioration, dementia, occurrence of ischemic events, occurrence of disability, migraine headaches, multiple strokes, seizures, vision problems and/or psychiatric problems such as depression, apathy and mood disturbances.

Vitamin E tocotrienols may constitute 10 to 40% of the composition, or more particularly 15 to 35% of the composition. In accordance with the preferred embodiment of the invention, the vitamin E tocotrienols can be any one or any combination of alpha-tocotrienol, beta-tocotrienol, gamma-tocotrienol, and delta-tocotrienol. Preferably, the composition further comprises alpha-tocopherol. In another embodiment of the invention, the composition may further comprise vitamin E tocopherols including, but not limited to, beta-tocopherol, gamma-tocopherol, and delta-tocopherol. Preferably, the vitamin E tocotrienols and tocopherols are derived from natural sources. More preferably, the vitamin E tocotrienols and tocopherols are derived from sources selected from the group consisting of palm oil, rice bran oil, annatto, and cereal grains such as barley, oat, rye and wheat germ.

Preferably, the composition disclosed herein is adapted for oral consumption. Vitamin E tocotrienols, and tocopherols, in the orally administered composition can reach the cerebrospinal fluid and brain. Vitamin E tocotrienols and tocopherols in the brain provide protection to the members of the nervous system against damage. In more particular, vitamin E tocotrienols and tocopherols target and inhibit the cSrc-regulated 12-lipoxygenase pathway which is known to be implicated in neurodegeneration associated with ischemic stroke. In the 12-lipoxygenase pathway, free arachidonic acid (AA) is cleaved and released from the membrane phospholipids by phospholipase, specifically phospholipase A2 during an ischemic event and hypoperfusion in the cerebrovascular network in the brain. Arachidonic acid is subsequently converted by 12-lipoxygenase to hydroperoxyeicosatetraenoic acid which is the key mediator of neurotoxicity, leading to neurodegeneration. Inhibition of the cSrc-regulated 12-lipoxygenase pathway by vitamin E tocotrienols and tocopherols protect against cerebrovascular hypoperfusion-induced injuries such as neurotoxicity and brain cell death, thus suggesting the ability of vitamin E tocotrienols and tocopherols to prevent or delay cognitive decline and dementia related to the death of brain neuron cells

In addition, arachidonic acid is highly susceptible to oxidative metabolism under pathologic conditions. AA that is cleaved from the phospholipid bilayer by phospholipase A₂ (PLA₂) can undergo uncontrolled oxidative metabolism, which is also known as AA cascade. Metabolism of AA amplifies the overall production of free radicals in the brain and subsequently causes oxidative damage to the brain tissues. vitamin E tocotrienols and tocopherols, however, are able to attenuate the AA cascade. In more particular, vitamin E tocotrienols and tocopherols inhibit the oxidative damage caused by free radicals generated during a pathologic condition.

Moreover, vitamin E tocotrienols and tocopherols are capable of preventing loss of white matter fiber tract connectivity after a stroke event by improving the cerebrovascular collateral circulation to the area of hypoperfusion in the brain by inducing arteriogenic tissue inhibitor of metalloprotease 1 expression to promote cerebrovascular arteriogenesis. This helps to improve blood circulation to the hypoperfusion sites in the brain. In addition, the composition is effective in mitigating injury present during cerebrovascular ischemic event in patients suffering from

CADASIL, in which the composition reduces stroke lesion volume, promotes vascular angiogenesis, and improves cerebrovascular collateral circulation. In one embodiment of the invention, the composition comprises alpha-tocotrienol in a concentration range of 3 to 20% by weight. In a more preferred embodiment of the invention, alpha-tocotrienol presents in the composition at a concentration ranging from 5 to 16% by weight of the composition. Beta-tocotrienol constitute 0.7 to 3% by weight of the composition, or more preferably 0.8 to 2% by weight of the composition. Gamma-tocotrienol is present at a concentration ranging from 6 to 30% by weight of the composition in one embodiment of the invention, or 8 to 25% by weight of the composition in a more preferred embodiment. Alternatively, delta-tocotrienol constitute 1.5 to 12% by weight of the composition, or more preferably 2 to 10% by weight of the composition. In one embodiment of the invention, 3 to 15% by weight of the composition is made up of alpha-tocopherol. In a more preferred embodiment, 5 to 12% by weight of the composition is made up of alpha-tocopherol.

As set forth in preceding description, vitamin E tocotrienols and tocopherols are present in the composition in conjunction with squalenes and pharmaceutically acceptable excipients. Preferably, squalene in the composition is plant-based squalene. Sources for squalene used in the composition include, but not limited to, olive, oil palm fruits, amaranth seed, and rice bran. Squalene in the composition is beneficial in preventing memory deterioration due to its anti-oxidant activity. Preferably, the composition disclosed herein comprises 2.5 to 10% of squalene by weight of the composition. More preferably, squalene constitutes 3 to 8.5% by weight of the composition.

Pharmaceutically acceptable excipients are compounds that are inert to the other ingredients and generally have no pharmacological effects. They are included in the composition disclosed herein for the purpose of long-term stabilization and/or enhancement of bioavailability of vitamin E tocotrienols, vitamin E tocopherols and squalene. The composition of the invention may comprise one or more pharmaceutically acceptable excipients selected from the group consisting of plant-based oil, water-based emulsifiers, oil-based emulsifiers, co-emulsifiers, antioxidants, and suspending agents. Preferably, pharmaceutically acceptable excipients is present at a concentration ranging from 0.1 to 50 % by weight of the composition. More preferably, pharmaceutically acceptable excipients is present at a concentration ranging from 0.15 to 40% by weight of the composition.

The composition described in the preceding description is preferably formulated into dosage forms including, but not limited to, capsules, tablets, emulsions, and suspensions. More preferably, the medicament is present in the dosage form of soft capsules for enhanced bioavailability. Administration of the medicament over a period of time ranging from 1 to 5 years can effectively result in slowing of the progressive neurodegenerative disease.

### Example

The following non-limiting example has been carried out to illustrate the preferred embodiments of the invention.

### Example 1

Case study of CADASIL patient progression under 24 months administering of the composition.

The composition of the present invention is capable of mitigating progression of abnormal brain lesions and reducing migraine headaches, without further occurrence of ischemic evens, disability, dementia, multiple strokes, seizures, vision problems or psychiatric problems, for at least 4 years from administering the composition.

**Table 1: Progression of white matter lesion volume in CADASIL patient over 2 years supplementation of the composition.**

| Assessment | Baseline | 6-month | 12-month | 24-month |
|---|---|---|---|---|
| White Matter Lesion (WML) volume (mm³) | 24,144 (100%) | 22,105 (92%) | 19,756 (82%) | 19,073 (79%) |
| Total Brain volume (mm³) | 1,240,930 | 1,237,441 | 1,200,312 | 1,203,070 |
| WML to Brain volume ratio | 1.94% | 1.79% | 1.65% | 1.59% |

**Table 2: Assessment of headache impact and cognitive function in CADASIL patient over 2 years supplementation of the composition.**

| Assessment | Baseline | 6-month | 12-month | 18-month | 24-month |
|---|---|---|---|---|---|
| Headache Impact Test (HIT-6) (Migraine ≥50) | 57 (substantial impact) | - | 36 (no impact) | 36 | 36 |
| Montreal Cognitive Assessment (Normal ≥26) | 29 | 30 | 26 | 27 | 28 |

### References

1. Ayata C (2010). CADASIL: Experimental insights from animal models. Stroke 41(suppl 1): S129 - S134.
2. Alves GS, Oliveira Alves CE, Lanna ME, Moreira DM, Engelhardt E and Laks J (2008). Subcortical ischemic vascular disease and cognition: A systemic review. Dementia & Neuropsychologia 2(2): 82 - 90.
3. André C (2010). CADASIL: pathogenesis, clinical and radiological findings and treatment. Arquivos de Neuro-Psiquiatria 68(2): 287 - 299.
4. Viswanathan A, Gschwendtner A, Guichard JP, Buffon F, Cumurciuc R, O'Sullivan M, Holtmannspötter M, Pachai C, Bousser MG, Dichgans M and Chabriat H (2007). Lacunar lesions are independently associated with disability and cognitive impairment in CADASIL. Neurology 69(2): 172 - 179.
5. Liem MK, van der Grond J, Haan J, van den Boom R, Ferrari MD, Knapp YM, Breuning MH, van Buchem MA, Middelkoop HAM and Lesnik Oberstein SAJ (2007). Lacunar infarcts are the main correlate with cognitive dysfunction in CADASIL. Stroke 38: 923 - 928.
6. Buffon F, Porcher R, Hernandez K, Kurtz A, Pointeau S, Vahedi K, Bousser MG and Chabriat H (2006). Cognitive profile in CADASIL. Journal of Neurology, Neurosurgery and Psychiatry 77(2): 175 - 180.
7. Opherk C, Peters N, Herzog J, Luedtke R and Dichgans M (2004). Long-term prognosis and causes of death in CADASIL: a retrospective study in 411 patients. Brain 127: 2533 - 2539.
8. Dichgans M, Markus HS, Salloway S, Verkkoniemi A, Moline M, Wang Q, Posner H and Chabriat HS (2008). Donepezil in patients with subcortical vascular cognitive impairment: a randomised double-blind trial in CADASIL. Lancet Neurology. 7: 310-318.
9. Khanna S, Roy S, Park HA and Sen CK (2007). Mechanisms of Signal Transduction: Regulation of c-Src Activity in Glutamate-induced Neurodegeneration. The Journal of Biological Chemistry 282: 23482 - 23490.
10. Khanna S, Parinandi NL, Kotha SR, Roy S, Rink C, Bibus D and Sen CK (2010). Nanomolar vitamin E α-tocotrienol inhibits glutamate-induced activation of phospholipase A2 and causes neuroprotection. Journal of Neurochemistry 112:1249 - 1260.
11. Sen CK, Rink C and Khanna S (2010). Palm oil-derived natural vitamin E α-tocotrienol in brain health and disease. Journal of the American College of Nutrition. 29(Suppl 3): 314S - 323S.
12. Rink C, Christoforidis G, Khanna S, Peterson L, Patel Y, Khanna S, Abduljalil A, Irfanoglu O, Machiraju R, Bergdall VK and Sen CK (2011). Tocotrienol vitamin E protects against preclinical canine ischemic stroke by inducing arteriogenesis. Journal of Cerebral Blood Flow & Metabolism 31(11): 2218 - 2230.

## Claims

1. A composition comprising vitamin E tocotrienols, squalenes, phytosterols and pharmaceutically acceptable excipients for use in mitigating debilitating effects of Cerebral Autosomal-Dominant Arteriopathy with Subcortical Infarcts and Leukoencephalopathy (CADASIL).

2. The composition for use according to claim 1, wherein the composition further comprises vitamin E tocopherols.

3. The composition for use according to claim 1 or 2, wherein the debilitating effects is any one or any combination of abnormal brain lesions, cognitive impairment, dementia, memory deterioration, occurrence of ischemic events, occurrence of disability, migraine headaches, multiple strokes, seizures, vision problems, depression, apathy and mood disturbances.

4. The composition for use according to any one of claims 1 to 3, wherein the vitamin E tocotrienol is any one or any combination of alpha-tocotrienol, beta-tocotrienol, gamma-tocotrienol, and delta-tocotrienol.

5. The composition for use according to any one of claims 1 to 4, wherein the vitamin E tocotrienols are present at a concentration ranging from 10 to 40 % by weight of the composition.

6. The composition for use according to any one of claims 1 to 5, wherein alpha-tocotrienol is present at a concentration ranging from 3 to 20 % by weight of the composition.

7. The composition for use according to any one of claims 1 to 6, wherein beta-tocotrienol is present at a concentration ranging from 0.7 to 3.0 % by weight of the composition.

8. The composition for use according to any one of claims 1 to 7, wherein gamma-tocotrienol is present at a concentration ranging from 6 to 30 % by weight of the composition.

9. The composition for use according to any one of 5 claims 1 to 8, wherein delta-tocotrienol is present at a concentration ranging from 1.5 to 12 % by weight of the composition.

10. The composition for use according to any one of claims 1 to 9, wherein alpha-tocopherol is present at a concentration ranging from 3 to 15 % by weight of the composition.

11. The composition for use according to any one of claims 1 to 10, wherein squalene is present at a concentration ranging from 2.5 to 10 % by weight of the composition.

12. The composition for use according to any one of claims 1 to 11, wherein the pharmaceutically acceptable excipient is present at a concentration ranging from 0.1 to 50 % by weight of the composition.

13. The composition for use according to any one of claims 1 to 12, wherein the pharmaceutically acceptable excipient is any one or any combination of plant-based oil, water-based emulsifiers, oil-based emulsifiers, co-emulsifiers, antioxidants, and suspending agents.

14. The composition for use according to any one of claims 1 to 13, wherein the composition is formulated into dosage form selected from the group consisting of capsule, tablet, emulsion and suspension.

## Patentansprüche

1. Zusammensetzung umfassend Vitamin-E-Tocotrienole, Squalene, Phytosterole und pharmazeutisch verträgliche Hilfsstoffe, zur Anwendung bei der Abmilderung schwächender Wirkungen der zerebralen autosomal-dominanten Arteriopathie mit subkortikalen Infarkten und Leukenzephalopathie (CADASIL).

2. Zusammensetzung zur Anwendung nach Anspruch 1, wobei die Zusammensetzung ferner Vitamin-E-Tocopherole umfasst.

3. Zusammensetzung zur Anwendung nach Anspruch 1 oder 2, wobei die schwächenden Wirkungen eines oder eine beliebige Kombination von abnormalen Hirnläsionen, kognitiver Beeinträchtigung, Demenz, Gedächtnisverschlechterung, Auftreten ischämischer Ereignisse, Auftreten von Behinderung, Migränekopfschmerzen, mehrfachen Schlaganfällen, Krampfanfällen, Sehstörungen, Depression, Apathie und Stimmungsstörungen sind.

4. Zusammensetzung zur Anwendung nach einem der Ansprüche 1 bis 3, wobei das Vitamin-E-Tocotrienol eines oder eine beliebige Kombination von Alpha-Tocotrienol, Beta-Tocotrienol, Gamma-Tocotrienol und Delta-Tocotrienol ist.

5. Zusammensetzung zur Anwendung nach einem der Ansprüche 1 bis 4, wobei die Vitamin-E-Tocotrienole in einer Konzentration im Bereich von 10 bis 40 Gew.-% der Zusammensetzung vorliegen.

6. Zusammensetzung zur Anwendung nach einem der Ansprüche 1 bis 5, wobei Alpha-Tocotrienol in einer Konzentration im Bereich von 3 bis 20 Gew.-% der Zusammensetzung vorliegt.

7. Zusammensetzung zur Anwendung nach einem der Ansprüche 1 bis 6, wobei Beta-Tocotrienol in einer Konzentration im Bereich von 0,7 bis 3,0 Gew.-% der Zusammensetzung vorliegt.

8. Zusammensetzung zur Anwendung nach einem der Ansprüche 1 bis 7, wobei Gamma-Tocotrienol in einer Konzentration im Bereich von 6 bis 30 Gew.-% der Zusammensetzung vorliegt.

9. Zusammensetzung zur Anwendung nach einem der Ansprüche 1 bis 8, wobei Delta-Tocotrienol in einer Konzentration im Bereich von 1,5 bis 12 Gew.-% der Zusammensetzung vorliegt.

10. Zusammensetzung zur Anwendung nach einem der Ansprüche 1 bis 9, wobei Alpha-Tocopherol in einer Konzentration im Bereich von 3 bis 15 Gew.-% der Zusammensetzung vorliegt.

11. Zusammensetzung zur Anwendung nach einem der Ansprüche 1 bis 10, wobei Squalen in einer Konzentration im Bereich von 2,5 bis 10 Gew.-% der Zusammensetzung vorliegt.

12. Zusammensetzung zur Anwendung nach einem der Ansprüche 1 bis 11, wobei der pharmazeutisch verträgliche Hilfsstoff in einer Konzentration im Bereich von 0,1 bis 50 Gew.-% der Zusammensetzung vorliegt.

13. Zusammensetzung zur Anwendung nach einem der Ansprüche 1 bis 12, wobei der pharmazeutisch verträgliche Hilfsstoff eines oder eine beliebige Kombination von pflanzlichem Öl, wasserbasierten Emulgatoren, ölbasierten Emulgatoren, Co-Emulgatoren, Antioxidantien und Suspendiermitteln ist.

14. Zusammensetzung zur Anwendung nach einem der Ansprüche 1 bis 13, wobei die Zusammensetzung in eine Darreichungsform formuliert ist, die aus der Gruppe ausgewählt ist bestehend aus Kapsel, Tablette, Emulsion und Suspension.

## Revendications

1. Composition comprenant des tocotriénols de vitamine E, des squalènes, des phytostérols et des excipients pharmaceutiquement acceptables, destinée à être utilisée pour atténuer les effets débilitants de l'artériopathie cérébrale autosomique dominante avec infarctus sous-corticaux et leucoencéphalopathie (CADASIL).

2. Composition destinée à être utilisée selon la revendication 1, où la composition comprend en outre des tocophérols de vitamine E.

3. Composition destinée à être utilisée selon la revendication 1 ou 2, où les effets débilitants sont l'un quelconque ou une combinaison quelconque parmi les lésions cérébrales anormales, les troubles cognitifs, la démence, la détérioration de la mémoire, l'apparition d'événements ischémiques, l'apparition d'un handicap, les migraines, les accidents vasculaires cérébraux multiples, les crises épileptiques, les troubles de la vision, la dépression, l'apathie et les troubles de l'humeur.

4. Composition destinée à être utilisée selon l'une quelconque des revendications 1 à 3, où le tocotriénol de vitamine E est l'un quelconque ou une combinaison quelconque parmi l'alpha-tocotriénol, le bêta-tocotriénol, le gamma-tocotriénol et le delta-tocotriénol.

5. Composition destinée à être utilisée selon l'une quelconque des revendications 1 à 4, où les tocotriénols de vitamine E sont présents à une concentration comprise entre 10 et 40 % en poids de la composition.

6. Composition destinée à être utilisée selon l'une quelconque des revendications 1 à 5, où l'alpha-tocotriénol est présent à une concentration comprise entre 3 et 20 % en poids de la composition.

7. Composition destinée à être utilisée selon l'une quelconque des revendications 1 à 6, où le bêta-tocotriénol est présent à une concentration comprise entre 0,7 et 3,0 % en poids de la composition.

8. Composition destinée à être utilisée selon l'une quelconque des revendications 1 à 7, où le gamma-tocotriénol est présent à une concentration comprise entre 6 et 30 % en poids de la composition.

9. Composition destinée à être utilisée selon l'une quelconque des revendications 1 à 8, où le delta-tocotriénol est présent à une concentration comprise entre 1,5 et 12 % en poids de la composition.

10. Composition destinée à être utilisée selon l'une quelconque des revendications 1 à 9, où l'alpha-tocophérol est présent à une concentration comprise entre 3 et 15 % en poids de la composition.

11. Composition destinée à être utilisée selon l'une quelconque des revendications 1 à 10, où le squalène est présent à une concentration comprise entre 2,5 et 10 % en poids de la composition.

12. Composition destinée à être utilisée selon l'une quelconque des revendications 1 à 11, où l'excipient pharmaceutiquement acceptable est présent à une concentration comprise entre 0,1 et 50 % en poids de la composition.

13. Composition destinée à être utilisée selon l'une quelconque des revendications 1 à 12, où l'excipient pharmaceutiquement acceptable est l'un quelconque ou une combinaison quelconque parmi de l'huile d'origine végétale, des émulsifiants à base d'eau, des émulsifiants à base d'huile, des co-émulsifiants, des antioxydants et des agents de suspension.

14. Composition destinée à être utilisée selon l'une quelconque des revendications 1 à 13, où la composition est formulée sous une forme posologique choisie dans le groupe constitué par une capsule, un comprimé, une émulsion et une suspension.
